(19) Europäisches Patentamt
European Patent Office
Office européen des brevets

(11) Publication number: **0 050 932**
**B1**

(12) # EUROPEAN PATENT SPECIFICATION

(45) Date of publication of patent specification: **21.03.84**

(21) Application number: **81304696.8**

(22) Date of filing: **09.10.81**

(51) Int. Cl.$^3$: **C 07 D498/04**, A 61 K 31/42, A 61 K 31/43 // (C07D498/04, 263/00, 205/00),(A61K31/43, 31/42)

(54) **Therapeutic compounds containing beta-lactams.**

(30) Priority: **24.10.80 GB 8034416**

(43) Date of publication of application: **05.05.82 Bulletin 82/18**

(45) Publication of the grant of the patent: **21.03.84 Bulletin 84/12**

(84) Designated contracting states: **BE CH DE FR IT LI NL**

(56) References cited:
**DE-A- 2 708 330**
**GB-A- 1 546 569**

(73) Proprietor: **BEECHAM GROUP PLC**
**Beecham House Great West Road**
**Brentford Middlesex (GB)**

(72) Inventor: **Brooks, Gerald**
**6 Worcester Road**
**Reigate Surrey, RH2 9HW (GB)**

(74) Representative: **Hesketh, Alan, Dr. et al**
**European Patent Attorney Beecham Pharmaceuticals**
**Great Burgh Yew Tree Bottom Road**
**Epsom, Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 050 932 B1

Jouve, 18, rue St-Denis, 75001 Paris, France

Therapeutic compounds containing β-lactams

This invention relates to a class of novel β-lactam compounds, to the process for their preparation and to pharmaceutical compositions containing them.

Belgian Patent No 850 779 discloses a class of compounds of the formula (A) :

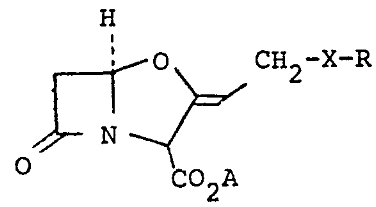

(A)

wherein X is S, SO or $SO_2$ and R is an organic group of up to 20 carbon atoms and A is a group such that $CO_2A$ represents a carboxylic acid group or a salt or ester thereof.

We have now found a novel class of compounds containing the thioacetal group, which have antibacterial and β-lactamase inhibitory activity.

Accordingly the present invention provides a compound of formula (I) or a pharmaceutically acceptable salt or *in vivo* hydrolysable ester thereof :

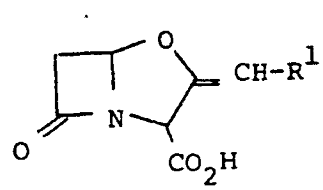

(I)

wherein $R^1$ is a group of structure (II) :

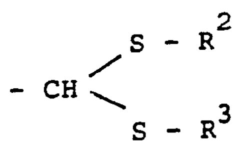

(II)

wherein $R^2$ and $R^3$ may be the same or different and each represents $C_{1-6}$ alkyl optionally substituted by $C_{1-6}$ alkoxy, phenyl, hydroxy, carboxy or $C_{1-6}$ alkoxycarbonyl, or phenyl optionally substituted by $C_{1-6}$ alkyl, halogen, $C_{1-6}$ alkoxy, hydroxy, carboxy or $C_{1-6}$ alkoxycarbonyl ;

or structure (III) :

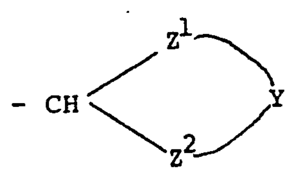

(III)

wherein $Z^1$, and $Z^2$ may be the same or different and each represents S, SO or $SO_2$ and Y represents a $C_{2-3}$ saturated hydrocarbon radical optionally substituted by $C_{1-6}$ alkyl, $C_{1-6}$ alkoxy, hydroxy, carboxy or $C_{1-6}$ alkoxycarbonyl.

Suitable groups $R^2$ and $R^3$ include methyl, ethyl, n- or iso-propyl, n-, sec-, tert- and iso-butyl, benzyl and phenyl.

Suitably the groups $R^2$ and $R^3$ are the same.

Suitable pharmaceutically acceptable salts of the compounds of formula (I) include metal salts, eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylamine, N,N-dibenzylethylenediamine, 1-ephenamine, N-ethylpiperidien, N-benzyl-β-phenethylamine, dehydroabietylamine, N,N'-bisdehydroabietylamine, ethylenediamine, or bases or the pyridine type such as pyridine, collidine or quinoline.

Examples of suitable pharmaceutically acceptable *in vivo* hydrolysable ester groups include those which break down readily in the human body to leave the parent acid or its salts, for example acyloxyalkyl

groups such as acetoxymethyl, pivaloyloxymethyl, α-acetoxyethyl and α-pivaloyloxymethyl groups ; alkoxycarbonyloxyalkyl groups, such as ethoxycarbonyloxymethyl ; dialkylamino alkyl groups such as dimethylaminomethyl, dimethylaminoethyl, diethylaminomethyl or diethylaminoethyl ; and α-ethoxycarbonyloxyethyl ; and lactone groups such as phthalidyl or dimethoxyphthalidyl.

One subgroup of compounds within the scope of this invention comprises compounds of formula (IV) or a pharmaceutically acceptable salt or *in vivo* hydrolysable ester thereof :

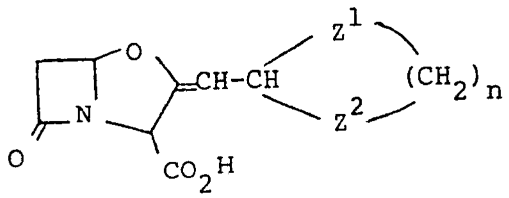

(IV)

wherein $Z^1$ and $Z^2$ are as defined hereinbefore with respect to formula (I), and n is an integer from 2 to 3.

Suitably n is 2.

Suitably $Z^1$ and $Z^2$ are the same and represent S or $SO_2$. Preferably $Z^1$ and $Z^2$ are both $SO_2$.

Suitable examples of $Z^1$ and $Z^2$ being different are $Z^1$ being S and $Z^2$ being SO.

The compounds of the present invention may be in the form of the Z-isomer, the E-isomer or a mixture of the E- and Z-isomer.

It will be appreciated that oxidation of compounds of formula (I) wherein Z is S may lead to a number of optically active isomers, and accordingly the present invention includes such isomers and mixtures thereof.

The present invention also provides a pharmaceutical composition which comprises a compound of this invention and a pharmaceutically acceptable carrier.

The compositions of the invention include those in a form adapted for oral, topical or parenteral use and may be used for the treatment of the infection in mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water ; sterile saline or the like and may also contain suspending agents such as polyvinylpyrrolidone, lecithin or the like. Alternatively such compositions may be prepared in an acceptable oil suspending agent such as arachis oil or its equivalent. For use in such suspensions the compounds of this invention should be in the form of fine particles.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the formula may be present in the composition as sole therapeutic agent or it may be present together with other therapeutic agents such as a penicillin or cephalosporin. Considerable advantages accrue from the inclusion of a penicillin or cephalosporin which shows instability to β-lactamases since the resulting composition shows enhanced effectiveness (synergy). Suitable penicillins cephalosporins or other β-lactam antibiotic for inclusion in such synergistic compositions include not only those known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, celbenicillin, pipericillin, and other known penicillins including pro-drugs thereof such as their *in vivo* hydrolysable esters such as the acetoxymethyl, pivaloyloxymethyl, α-ethoxycarbonyloxyethyl or phthalidyl esters of ampicillin, benzylpenicillin or amoxycillin, and aldehyde or ketone adducts of penicillins containing a 6-α-aminoacetamide side chain (such as hetacillin, metampicillin and analogous derivatives of amoxycillin) or α-esters of carbenicillin or ticarcillin such as their phenyl or indanyl α-esters.

Suitable cephalosporins for inclusion in the compositions of this invention include cefatrizine, cephaloridine, cephalothin, cefazolin, cephalexin, cephacetrile, cephamandole nafate, cephapirin, cephradine, 4-hydroxycephalexin, cefaparole, cephaloglycin, cefoperazone, and other known cephalosporins or produgs thereof.

Such compounds are frequently used in the form of a salt or hydrate of the like.

Naturally if the penicillin or cephalosporin present in the composition is not suitable for oral administration then the composition will be adapted for parenteral administration.

Highly favoured penicillins for use in the compositions of this invention include ampicillin, amoxycillin, carbenicillin and ticarcillin. Such penicillins may be used as a pharmaceutically acceptable salt such as the sodium salt. Alternatively the ampicillin or amoxycillin may be used in the form of fine particles of the zwitterionic form (generally as ampicillin trihydrate or amoxycillin trihydrate) for use in an injectable suspension, for example, in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt for example the sodium salt.

When present together with a cephalosporin or penicillin, the ratio of a compound of the invention to the penicillin or cephalosporin agent may vary over a wide range of ratios, such as from 10 : 1 to 1 : 10 for example about 3 : 1, 2 : 1, 1 : 1, 1 : 2, 1 : 3, 1 : 4, 1 : 5 or 1 : 6, (wt/wt, based on pure free antibiotic equivalent). Orally administrable compositions containing a compound of the invention will normally contain relatively more synergist than corresponding injectable compositions.

The total quantity of a compound of the invention in any unit dosage form will normally be between 25 and 1,000 mg and will usually be between 50 and 500 mg, for example about 62.5, 100, 125, 150, 200 or 250 mg.

Compositions of this invention may be used for the treatment of infections of *inter alia,* the respiratory tract, the urinary tract and soft tissues in humans and mastitis in cattle.

Normally between 50 and 3,000 mg of the compounds of the invention will be administered each day of treatment but more usually between 100 and 1,000 mg of the compounds of the invention will be administered per day, for example at 1-6 doses, more usually as 2, 3 or 4 doses. However for the treatment of more severe systemic infections or infections of particularly intransigent organisms higher doses may be used in accordance with clinical practice.

The penicillin or cephalosporin in the synergistic composition of this invention will normally be present at approximately the amount at which it is conventionally used which will usually be expected to be from about 62.5 to 3,000 mg per dose, more usually about 125, 250, 500 or 1,000 mg per dose.

One particularly favoured composition of this invention will contain from 150 to 1,000 mg of amoxycillin as the trihydrate or sodium salt and from 25 to 500 mg of a compound of this invention.

A further particularly favoured composition of this invention will contain from 150 to 1,000 mg of ampicillin or a pro-drug thereof and from 25 to 500 mg of a compound of this invention.

Most suitably this form of composition will contain ampicillin trihydrate, ampicillin anhydrate, sodium ampicillin, hetacillin, pivampicillinhydrochloride, bacampicillin hydrochloride, or talampicillin hydrochloride. Most suitably this form of the composition will contain a compound of the formula (I) when in crystalline form.

Most suitably the preceding composition will contain from 200 to 700 mg of the penicillin component. Most suitably the preceding composition will comprise from 50 to 250 mg of a compound of the formula (I) preferably in crystalline form.

Such compositions may be adapted for oral or parenteral use except when containing an *in vivo* hydrolysable ester of ampicillin or amoxycillin in which case the compositions will not be adapted for parenteral administration.

Another particularly favoured composition of this invention will contain from 200 to 2,000 mg of carbenicillin, ticarcillin or a pro-drug thereof and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of the formula (I) preferably in crystalline form. Such compositions containing di-salts of carbenicillin and ticarcillin will be adapted for parenteral administration.

The present invention also provides a composition for use is treating bacterial infections in humans or domestic mammals.

Commonly the infection treated will be due to a strain of *Staphylococcus aureus, Klebsiella aerogenes, Escherichia coli, Proteus sp., Bacteroides fragilis* or the like. The organisms believed to be most readily treated by an antibacterially effective amount of a compound of this invention is *Staphylococcus aureus.* The other organisms named are more readily treated by using a synergistically effective amount of the compound of the invention and a penicillin or cephalosporin. The administration of the two components may take place separately but in general we prefer to use a composition

containing both the synergist and the penicillin or cephalosporin.

The indications for treatment include respiratory tract and urinary tract infections in humans and mastitis in cattle.

The present invention also provides a process for the preparation of a compound of formula (I) or a pharmaceutically acceptable salt or *in vivo* hydrolysable ester thereof which process comprises reacting a compound of formula (V) :

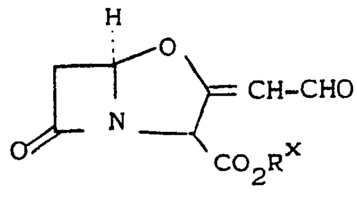

(V)

wherein $R^x$ is a carboxyl blocking group ;

1) with compounds $R^2SH$ and $R^3SH$ wherein $R^2$ and $R^3$ are as defined with respect to formula (I) and wherein any reactive groups may be protected ; or

2) with a compound of formula HS-Y-SH wherein Y is as defined with respect to formula (I) and wherein any reactive groups may be protected ; in the presence of a Lewis acid catalyst ; and thereafter where necessary carrying out one or more of the following steps ;

(a) oxidising a sulphide to a sulphoxide or sulphone derivative ;

(b) removing the carboxy blocking group $R^x$ ;

(c) removing any protecting groups on $R^2$ or $R^3$, or Y ; and

(d) converting the compound to a free carboxylic acid, a pharmaceutically acceptable salt or *in vivo* hydrolysable ester.

It will be appreciated that when $R^2$ and $R^3$ are the same in formula (I) then compounds $R^2SH$ and $R^3SH$ will also be the same, and when $R^2$ and $R^3$ are different in formula (I) then a mixture of compounds $R^2SH$ and $R^3SH$ will be used.

The starting material of formula (V) is disclosed in UK Patent No 1 504 426.

Suitable carboxyl-blocking derivatives for the group $—CO_2R^x$ in formula (V) include ester derivatives of the carboxylic acid. The derivative is preferably one which may readily be cleaved at a later stage of the reaction.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl 4-hydroxybenzyl, benzoylmethyl, p-nitrobenzyl, 4-pyridylmethyl, 2,2,2-trichloroethyl, 2,2,2-tribromoethyl, allyl, diphenylmethyl, triphenylmethyl, 2-benzyloxyphenyl, 4-methylthiophenyl, methoxymethyl, a silyl, stannyl or phosphorus-containing group, or an *in vivo* hydrolysable ester radical such as defined above.

The carboxylic group or a salt thereof may be regenerated from any of the above esters by usual methods appropriate to the particular $R^x$ group, for example, base-catalysed hydrolysis, or by enzymically-catalysed hydrolysis, or by hydrogenation.

Suitable Lewis acid catalysts include boron trifluoride or its equivalent such as a boron trifluoride etherate, for example $BF_3 \cdot O(C_2H_5)_2$.

The preceding reaction normally takes place in a solvent inert under the reaction conditions such as chloroform, dichloromethane, tetrahydrofuran or dioxane or may be carried out using the thiol or dithiol reagent as solvent. The reaction is generally carried out at a depressed or non-elevated temperature, for example − 80 °C to + 30 °C, and preferably at a depressed temperature, for example − 20 °C to 0 °C, and conveniently at about 0 °C.

Those compounds of formula (I) wherein Z is SO or $SO_2$ can be prepared from the corresponding compound wherein Z is S by mild oxidation.

Such reactions may take place at an ambient or depressed temperature, for example at − 20 °C to + 20 °C, more suitably at − 12 °C to + 5 °C, for example at about 0 °C.

The oxidation is best brought about using an organic per-acid as the oxidizing agent. Suitable acids include m-chloroperbenzoic acid and equivalent reagents. Use of two equivalents of the oxidizing agent leads to a compound of the formula (I) wherein Z is SO whereas the use of an excess of the oxidising agent leads to a compound of the formula (I) wherein Z is $SO_2$. The use of one equivalent of the oxidising agent leads to compounds wherein only one sulphur has been oxidised to the sulphoxide.

It is normal to carry out the oxidation in an inert solvent such as methylene chloride or the like.

Acids within formula (I) may be prepared by the careful acidification of a corresponding salt such as the sodium salt.

Salts within formula (I) may also be prepared by salt exchange in conventional manner, for example a solution of the lithium salt in water may be passed through a bed of ion exchange resin in the sodium form (eg Amberlite 120 (Trade name) ; a sodium salt of a sulphonated polystyrene divinyl benzene co-polymer) in about ten-fold excess until elution is complete ; the resulting sodium salt may be obtained by freeze drying or the like. Similarly a sodium salt may be converted to a lithium salt or to a potassium salt in

similar manner.

The following Examples illustrate the invention.

## Example 1

Benzyl 2-E-(2',2'-diethylthioethylidene)clavam-3-carboxylate and benzyl 2-Z-(2',2'-diethylthioethylidene)clavam-3-carboxylate

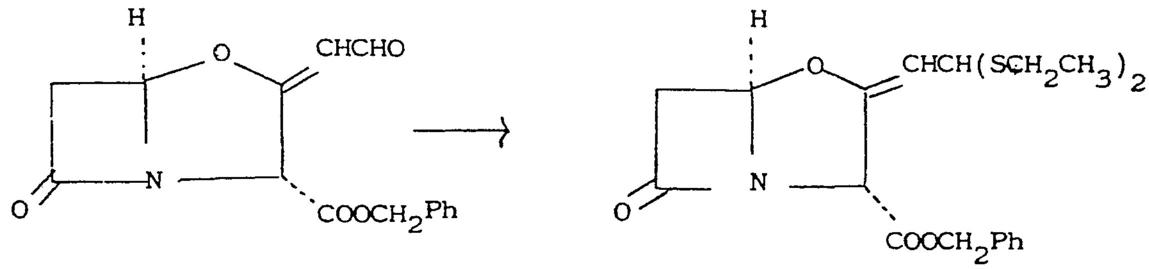

(e1) (e2) and (e3)

A mixture of E and Z isomers of benzyl 2-(2'-oxoethylidene)clavam-2-carboxylate (e1) (150 mg, 0.523 mmole) was dissolved in ethane thiol (5 ml). Boron trifluoride diethyletherate (1 drop) was added, the mixture stirred for 30 minutes and diluted with ethyl acetate (20 ml). This solution was washed with 1N aqueous sodium bicarbonate solution (20 ml), dried over anhydrous magnesium sulphate and evaporated under reduced pressure. The residue was chromatographed on silica (30 g, Merck Kieselgel 60 (Trade name), 230-400 mesh, in a 3 cm diameter column), eluting with ethyl acetate/petrol 1 : 4. The title compounds (e2) and (e3) were detected on t. l. c. (silica, eluent ethyl acetate/petrol 1 : 1) by spraying the plates with dilute potassium permanganate solution. Less polar isomer (e2) (colourless gum, 70 mg, 34 %) : $[\alpha]_D^{20}$ – 32.0° (c. 0.7 ; $CHCl_3$) ; $\nu_{max}$ ($CHCl_3$) 1,800, 1,745 and 1,680 $cm^{-1}$ ; δ ($CDCl_3$) 1.18 and 1.22 (6H, 2t, —$SCH_2CH_3$), 2.55 (4H, broad q, —$SCH_2CH_3$), 3.01 (1H, d, J 17 Hz, 6β-CH), 3.46 (1H, dd, J 17 and 2.5 Hz, 6α-CH), 4.56 (1H, d, J 12 Hz, 8-CH or 9-CH), 5.0-5.4 (4H, m, —$OCH_2Ph$, 3-CH and 8-CH or 9-CH), 5.68 (1H, d, J 2.5 Hz, 5-CH), 7.35 (5H, s, Ph-H).

More polar isomer (e3) (colourless gum, 40 mg, 19 %) : $[\alpha]_D^{20}$ + 5.6° (c. 1.3 ; $CHCl_3$) ; $\nu_{max}$ ($CHCl_3$) 1,800, 1,750 and 1,680 $cm^{-1}$ ; δ ($CDCl_3$) 1.18 and 1.24 (6H, 2t, —$SCH_2CH_3$), 2.47 and 2.59 (4H, 2q, —$SCH_2CH_3$), 3.01 (1H, d, J 17 Hz, 6β-CH), 3.48 (1H, dd, J 17 and 2.5 Hz, 6α-CH), 4.78 (2H, s, 8-CH and 9-CH), 5.10 (1H, s, 3-CH), 5.18 (2H, s, —$OCH_2Ph$), 5.68 (1H, d, J 2.5 Hz, 5-CH), 7.34 (5H, s, Ph-H).

## Example 2

p-Nitrobenzyl 2-E-(2',2'-diethylthioethylidene)clavam-3-carboxylate and p-nitrobenzyl 2-Z-(2',2'-diethylthioethylidene)clavam-3-carboxylate

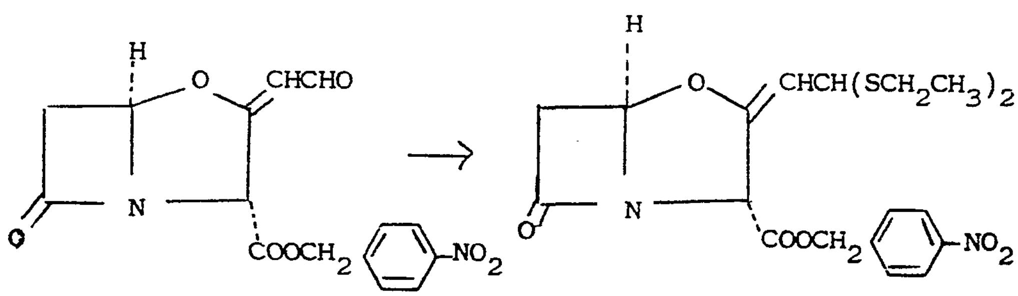

(e4) (e5) and (e6)

A mixture of E and Z isomers of p-nitrobenzyl 2-(2'-oxoethylidene)clavam-3-carboxylate (e4) (700 mg, 2.11 mmole) was dissolved in ethanethiol (8 ml), ice cooled and boron trifluoride diethyl etherate (3 drops) added. The mixture was stirred for 45 minutes at 0-5 °C and diluted with dichloromethane (30 ml). The resulting solution was washed with 1N aqueous sodium bicarbonate solution (30 ml), dried over anhydrous magnesium sulphate and evaporated under reduced pressure. This residue was chromatographed on silica (35 g), eluent ethyl acetate/petrol 1 : 4, in a similar manner to that described in Example 1 to provide two compounds (e5) and (e6) with $R_f$'s of 0.72 and 0.67 respectively on t. l. c. (silica, eluent ethyl acetate/petrol 1 : 1).

Less polar isomer (e5) (colourless gum, 230 mg, 28 %) : $[\alpha]_D^{20}$ – 30.8° (c. 1.2 ; $CHCl_3$) ; $\nu_{max}$ ($CHCl_3$) 1,800, 1,750 and 1,680 $cm^{-1}$ ; δ ($CDCl_3$) 1.17 and 1.23 (6H, 2t, —$SCH_2CH_3$), 2.4-2.8 (4H, m, —$SCH_2CH_3$),

3.04 (1H, d, J 17 Hz, 6β-CH), 3.48 (1H, dd, J 17 and 2.5 Hz, 6α-CH), 4.52 (1H, d, J 11 Hz, 8-CH or 9-CH), 5.1-5.5 (4H, m, —$OCH_2Ar$, 3-CH, 8-CH or 9-CH), 5.68 (1H, d, J 2.5 Hz, 5-CH), 7.52 and 8.23 (4H, 2d, Ar-H) ; Found 438.0885 ($M^+$) ; $C_{19}H_{22}N_2O_6S_2$ requires 438.0919.

More polar isomer (e6) (colourless gum, 195 mg, 23 %) : $[\alpha]_D^{20}$ + 5.9° (c. 1.1 ; $CHCl_3$) ; $\nu_{max}$ ($CHCl_3$) 1,800, 1,750 and 1,680 $cm^{-1}$ ; δ ($CDCl_3$) 1.20 and 1.24 (6H, 2t, —$SCH_2CH_3$), 2.52 and 2.60 (4H, 2q, —$SCH_2CH_3$), 3.04 (1H, d, J 17 Hz, 6β-CH), 3.51 (1H, dd, J 17 and 2.5 Hz, 6α-CH), 4.78 (2H, s, 8-CH and 9-CH), 5.14 (1H, s, 3-CH), 5.29 (2H, ABq, —$OCH_2Ar$), 5.69 (1H, d, J 2.5 Hz, 5-CH), 7.51 and 8.23 (4H, 2d, Ar-H) ; Found 438.0925($M^+$) ; $C_{19}H_{22}N_2O_6S_2$ requires 438.0919.

## Example 3

Mixture of E and Z isomers of p-nitrobenzyl 2-(2′,2′-diethylthioethylidene)clavam-3-carboxylate

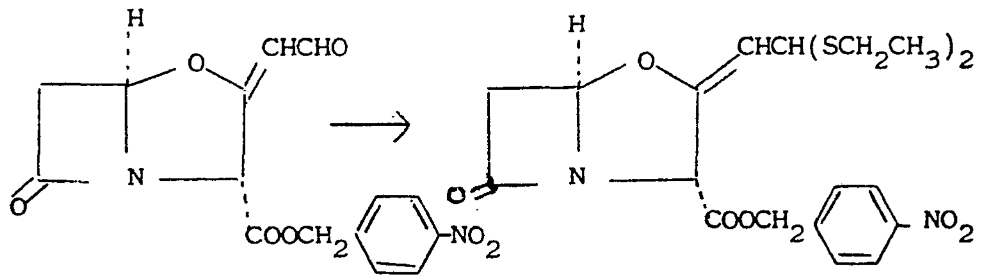

(e4) (e5) and (e6)

A mixture of E and Z isomers of p-nitrobenzyl 2-(2′-oxoethylidene)clavam-3-carboxylate (e4) (185 mg, 0.558 mmole) was dissolved in a mixture of dichloromethane (3 ml) and ethanethiol (2 ml), ice cooled and treated with boron trifluoride diethyletherate (3 drops). The mixture was stirred 45 minutes at 0-5 °C, diluted with dichloromethane (10 ml), washed with 1N aqueous sodium bicarbonate solution (20 ml), dried over anhydrous magnesium sulphate and evaporated under reduced pressure. Chromatography of the residue as described in Example 1, eluting with ethyl acetate/petrol 1 : 3, provided a mixture of E and Z isomers of p-nitrobenzyl 2-(2′,2′-diethylthioethylidene)clavam-3-carboxylate (e5) and (e6) (0.12 g, 49 %).

## Example 4

Lithium 2-(2′,2′-diethylthioethylidene)clavam-3-carboxylate (less polar isomer)

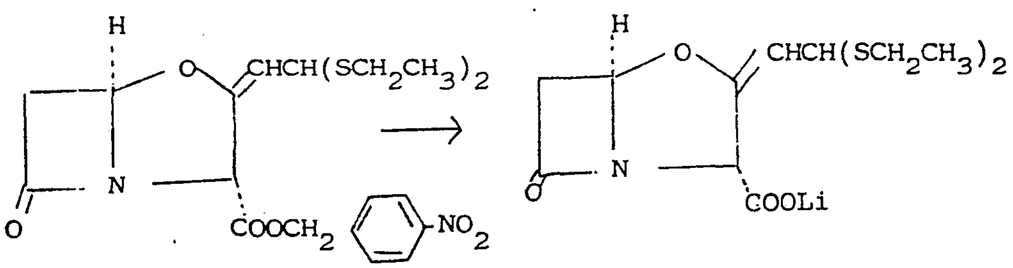

(e5) (e7)

A solution of p-nitrobenzyl 2-(2′,2′-diethylthioethylidene)clavam-3-carboxylate (e5) (less polar product of Example 2, 120 mg, 0.274 mmole) in tetrahydrofuran (5 ml) was added to a pre-hydrogenated suspension of 10 % Pd/C (180 mg) in tetrahydrofuran (5 ml). Hydrogenolysis was continued at atmospheric pressure for 1 1/4 hours, the suspension filtered and the filtrate evaporated to 2 ml under reduced pressure. With ice cooling, water (5 ml) and 0.1M aqueous lithium carbonate solution (1.37 ml) were added. The resulting solution was washed with ethyl acetate (3 × 10 ml), adjusted to pH 7 with 0.1N hydrochloric acid and evaporated to dryness under reduced pressure. The residue was chromatographed on cellulose (15 g in a 2.5 cm diameter column, Whatman CC31), eluting with n-butanol/ethanol/water 4 : 1 : 1. Appropriate fractions were combined and evaporated to provide lithium 2-(2′,2′-diethylthioethylidene)clavam-3-carboxylate (e7) (30 mg, 24 %, less polar isomer on a silica t. l. c., eluting with chloroform/acetone/glacial acetic acid 50 : 50 : 7) as a yellow solid. $[\alpha]_D^{20}$-13.1° (c. 0.5 ; DMSO) ; $\nu_{max}$ (KBr)

1,785, 1,680 and 1,620 $cm^{-1}$ ; δ ($D_2O$—HOD=4.60) 1.18 and 1.20 (6H, 2t, —$SCH_2CH_3$), 2.58 and 2.62 (4H, 2q, —$SCH_2CH_3$), 3.03 (1H, d, J 17 Hz, 6β-CH), 3.53 (1H, dd, J 17 and 2.5 Hz, 6α-CH), 4.82 (1H, d, J 11.5 Hz, 9-CH), 4.9-5.2 (2H, m, 3-CH and 8-CH), 5.73 (1H, d, J 2.5 Hz, 5-CH).

## Example 5

Lithium 2-(2′,2′-diethylthioethylidene)clavam-3-carboxylate (more polar isomer)

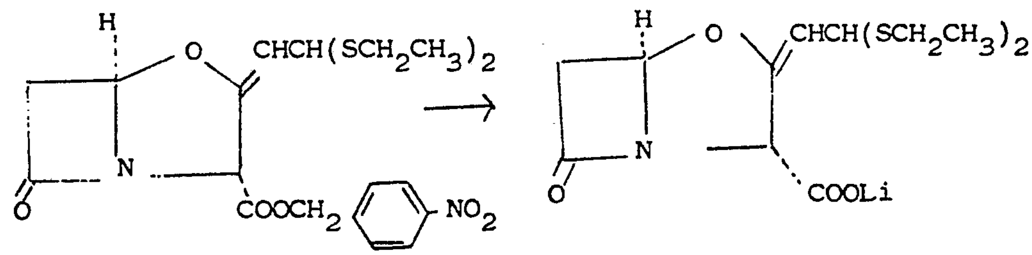

(e6) (e8)

A solution of p-nitrobenzyl 2-(2′,2′-diethylthioethylidene)clavam-3-carboxylate (e6) (more polar product of Example 2, 100 mg, 0.228 mmole) in tetrahydrofuran (5 ml) was added to a pre-hydrogenated suspension of 10% Pd/C (150 mg) in tetrahydrofuran (5 ml). Hydrogenolysis was carried out at atmospheric pressure for 1 1/4 hours, the mixture filtered and the filtrate evaporated to 2 ml under reduced pressure. With ice cooling, water (5 ml) and 0.1M aqueous lithium carbonate solution (1.14 ml) were added. The resulting solution was washed with ethyl acetate (3 × 10 ml), adjusted to pH7 with 0.1N hydrochloric acid and evaporated to dryness under reduced pressure. The residue was chromatographed as described in Example 4 to provide lithium 2-(2′,2′-diethylthioethylidene)clavam-3-carboxylate (e8) (38 mg, 53%, more polar isomer on silica t. l. c., eluting with chloroform/acetone/glacial acetic acid 50 : 50 ; 7) as a yellow solid. $[\alpha]_D^{20}$ + 19.8° (c. 0.5 ; DMSO) ; $\nu_{max}$ (KBr) 1,785, 1,685 and 1,620 $cm^{-1}$ ; δ ($D_2O$—HOD=4.60) 1.18 and 1.20 (6H, 2t, —$SCH_2CH_3$), 2.58 and 2.63 (4H, 2q, —$SCH_2CH_3$), 3.04 (1H, d, J 17 Hz, 6β-CH), 3.54 (1H, dd, J 17 and 2.5 Hz, 6α-CH), 4.6-5.1 (3H, m, 8-CH, 9-CH and 3-CH), 5.70 (1H, d, J 2.5 Hz, 5-CH).

## Example 6

Mixture of E and Z isomers of benzyl 2-(1′,3′-dithiolan-2′-yl methylene)clavam-3-carboxylate

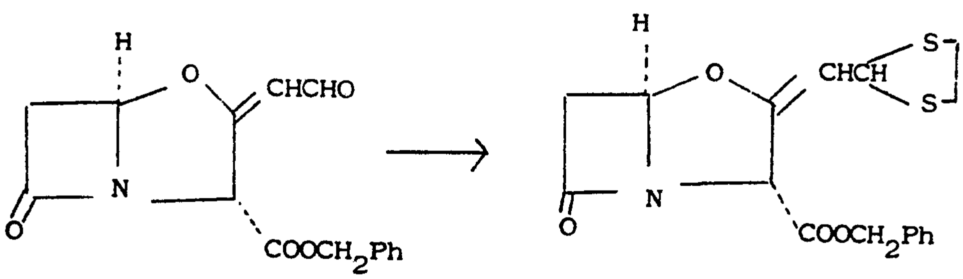

(e1) (e9)

A mixture of E and Z isomers of benzyl 2-(2′-oxoethylidene)clavam-3-carboxylate (e1) (800 mg, 2.79 mmole) was dissolved in ethane-1,2-dithiol (5 ml), ice cooled and treated with borontrifluoride diethyl etherate (2 drops). The mixture was stirred at 0-5 °C for 1 hour, diluted with dichloromethane (100 ml), washed with 1N aqueous sodium bicarbonate solution (50 ml), dried over magnesium sulphate and run on to a silica column (40 g of silica on a 3 cm diameter column). Chromatography was carried out in the manner of Example 1, eluting with ethyl acetate/petrol (15% ethyl acetate), to provide an approximately 1 : 1 mixture of E and Z isomers of benzyl 2-(1′,3′-dithiolan-2′-ylmethylene)clavam-3-carboxylate (e9) (520 mg, 51%) as a colourless gum. $[\alpha]_D^{20}$ + 11.5° (c. 1.3 ; $CHCl_3$) ; $\nu_{max}$ ($CHCl_3$) 1,800, 1,750 and 1,680 $cm^{-1}$. δ ($CDCl_3$) 3.00 and 3.04 (1H, 2d, J 17 Hz, 6β-CH), 3.1-3.6 (5H, m, —$SCH_2CH_2S$— and 6α-CH), 4.86 (broad d, J 10.5 Hz, 8-CH of isomer A), 5.04 (broad s, 3-CH of isomer A), 5.1-5.4 (m, —$OCH_2Ph$ and 3-CH, 8-CH, 9-CH of isomer B), 5.46 (d, J 10.5 Hz, 9-CH of isomer A), 5.6-5.8 (1H, m, 5-CH), 7.35 (5H, s, Ph-H).

N.B. The Letters A and B are assigned arbitrarily and do not define which isomer is E or Z.

Example 7

Mixture of E and Z isomers of benzyl 2-(1′,3′-dithiolan-2′-ylmethylene)clavam-3-carboxylate

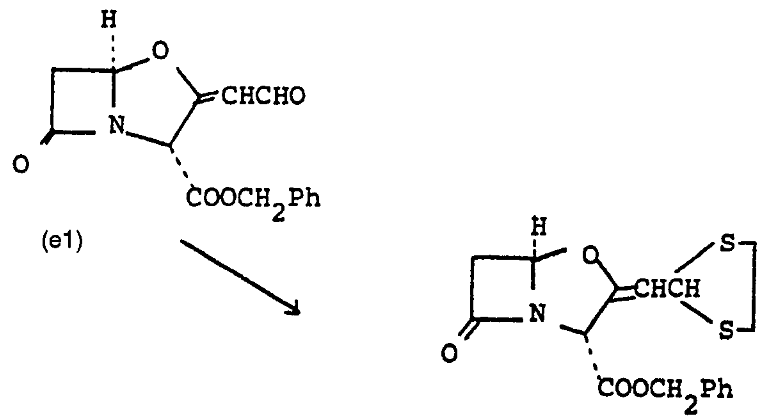

(e1)

(e9)

A mixture of E and Z isomers of benzyl 2-(2′-oxoethylidene)clavam-3-carboxylate (e1) (500 mg, 1.74 mmole) was dissolved in a mixture of dichloromethane (3 ml) and ethane-1,2-dithiol (2 ml), ice cooled and treated with boron trifluoride diethyl etherate (3 drops). The mixture was stirred at 0-5 °C for 45 minutes and worked up exactly as described in Example 6, to provide a mixture of E and Z isomers of benzyl 2-(1′,3′-dithiolan-2′-ylmethylene)clavam-3-carboxylate (e9) (290 mg, 46 %), identical on t. l. c. and spectroscopically to the product of Example 6.

Example 8

Mixture of E and Z isomers of benzyl 2-(1′,3′-dithiolan-2′-ylmethylene)clavam-3-carboxylate

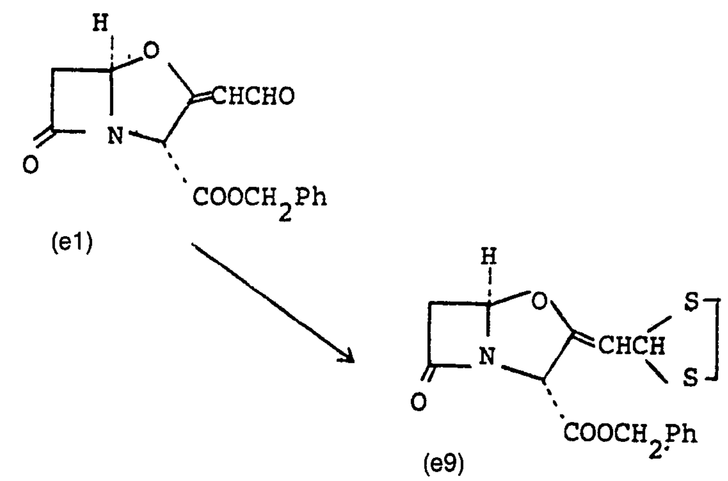

(e1)

(e9)

A mixture of E and Z isomers of benzyl 2-(2′-oxoethylidene)clavam-3-carboxylate (e1) (150 mg, 0.523 mmole) was dissolved in ethane-1,2-dithiol (3 ml) and pyridine hydrochloride (5 mg) was added. The mixture was stirred 2 days at room temperature, diluted with dichloromethane (50 ml), washed with water (50 ml) and dried over magnesium sulphate. The resulting solution was added to a silica column and chromatographed as described in Example 6 to provide a mixture of E and Z isomers of benzyl 2-(1′,3′-dithiolan-2′-ylmethylene)clavam-3-carboxylate (e9) (70 mg, 37 %). NMR spectroscopy showed the geometric isomers to be present in a ratio of approximately 85 : 15, and by t. l. c. and IR the product was identical to that of Example 6. $[\alpha]_D^{20}$ + 3.2° (c. 1.26 ; $CHCl_3$).

Example 9

Mixture of E and Z isomers of p-nitrobenzyl 2-(1′,3′-dithiolan-2′-ylmethylene)clavam-3-carboxylate

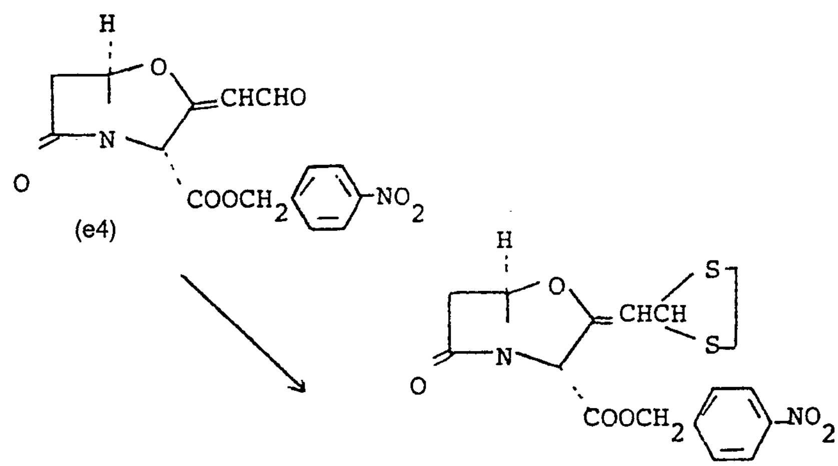

(e10)

A mixture of E and Z isomers of p-nitrobenzyl 2-(2′-oxoethylidene)clavam-3-carboxylate (e4) (700 mg, 2.11 mmole) was dissolved in ethane-1,2-dithiol (5 ml), ice cooled and treated with boron trifluoride diethyl etherate (3 drops). The mixture was stirred at 0-5 °C for 45 minutes, diluted with dichloromethane (100 ml), washed with 1N aqueous sodium bicarbonate solution (50 ml) and dried over magnesium sulphate. The resulting solution was run on to a silica column (40 g of silica in a 3 cm diameter column) and chromatographed in the manner of Example 1, eluting with ethyl acetate/petrol (30 % of ethylacetate) to provide a mixture of E and Z isomers of p-nitrobenzyl 2-(1′,3′-dithiolan-2′-ylmethylene)clavam-3-carboxylate (e10) (430 mg, 50 %). $[\alpha]_D^{20}$ − 4.9° (c. 1.2 ; $CHCl_3$) ; $\nu_{max}$ ($CHCl_3$) 1,805, 1,755 and 1,680 $cm^{-1}$ ; $\lambda_{max}$ (EtOH) 264 nm (13310) ; δ ($CDCl_3$) 2.9-3.7 (6H, m, 6-$CH_2$ and —$SCH_2CH_2S$—), 4.83 (dd, J 10 and 1 Hz, 8-CH of one isomer), 5.0-5.5 (m, —$OCH_2Ar$, 3-CH, 9-CH, and 8-CH of one isomer), 5.6-5.8 (1H, m, 5-CH), 7.50 and 7.54 (2H, 2d, 2 Ar-H), 8.23 (2H, d, 2 Ar-H) ; Found 408.0474 ($M^+$) : $C_{17}H_{16}N_2O_6S_2$ requires 408.0447.

Example 10

Mixture of E and Z isomers of lithium 2-(1′,3′-dithiolan-2′-ylmethylene)clavam-3-carboxylate

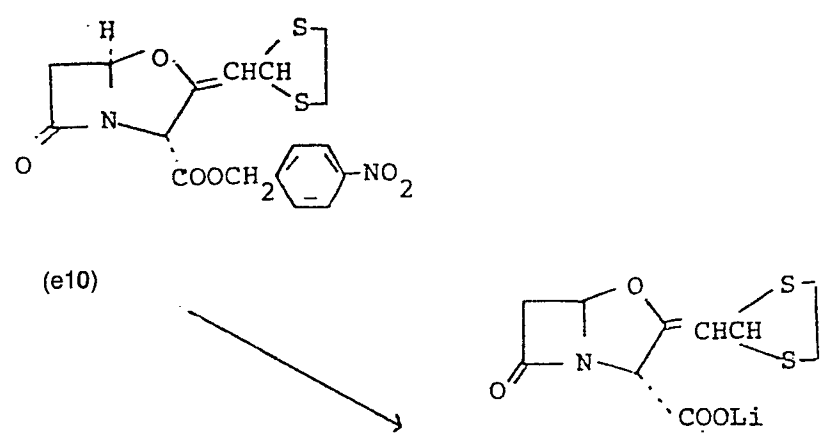

(e11)

A solution of a mixture of E and Z isomers of p-nitrobenzyl 2-(1′,3′-dithiolan-2′-ylmethylene)clavam-3-carboxylate (e10) (170 mg, 0.416 mmole) in tetrahydrofuran (5 ml) was added to a pre-hydrogenated suspension of 10 % Pd/c in tetrahydrofuran (5 ml). Hydrogenolysis was carried out at atmospheric pressure for 1 1/2 hours and the mixture filtered through celite (Trade name). The filtrate was evaporated to 2 ml, treated with water (5 ml) and 0.1M aqueous lithium carbonate solution (2.13 ml), and washed with ethyl acetate (3 × 10 ml). The aqueous solution was then saturated with sodium chloride, acidified to pH 2 with 1N hydrochloric acid and extracted with ethyl acetate (3 × 10 ml). The combined organic extracts were dried over magnesium sulphate and evaporated under reduced pressure. The residue was taken up in a mixture of tetrahydrofuran (2 ml) and water (2 ml) and brought to pH7 with 0.1M aqueous lithium carbonate solution. Organic solvent was removed under reduced pressure and the remaining aqueous solution freeze dried to provide a mixture of E and Z isomers of lithium 2-(1′,3′-dithiolan-2′-

ylmethylene)clavam-3-carboxylate (e11) (50 mg, 32 %) as a yellow solid. $[\alpha]_D^{20}$ + 22.5° (c. 0.7 ; $H_2O$) ; $\nu_{max}$ (KBr) 1,785, 1,680 and 1,620 $cm^{-1}$ ; δ ($D_2O$—$CH_3CN$=2.00) 2.99 and 3.04 (1H, 2d, J 17.5 Hz, 6β-CH), 3.1-3.7 (5H, m, 6α-CH and —$SCH_2CH_2S$—), 4.83 and 5.12 (1H, 2d, J 1.5 Hz, 3-CH), 4.84 (dd, J 10.5 and 1.5 Hz) and 5.11 (dd, J 11.5 and 1.5 Hz) (1H, 8-CH), 5.40 (d, J 11.5 Hz) and 5.47 (d, J 10.5 Hz) (1H, 9-CH), 5.6-5.7 (1H, m, 5-CH).

Example 11

Mixture of E and Z isomers of p-nitrobenzyl 2-(1',1',3',3'-tetraoxido-1',3'-dithiolan-2'-ylmethylene)clavam-3-carboxylate

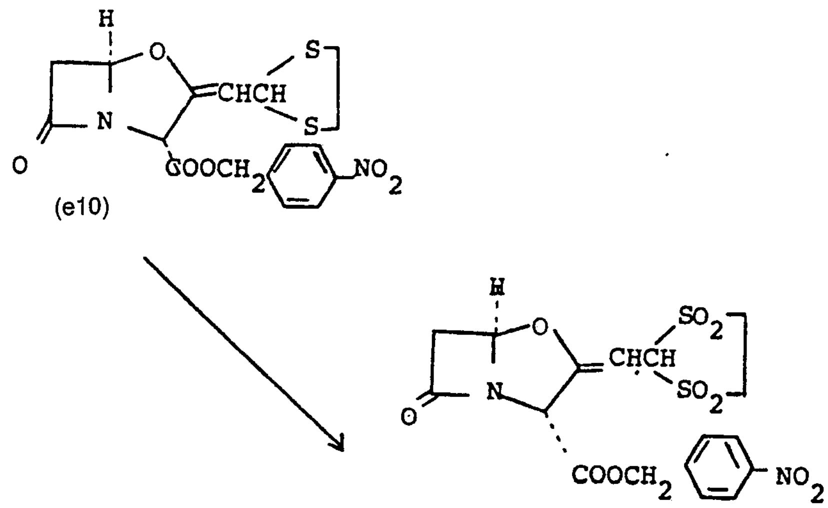

(e10)

(e12)

A solution of a mixture of E and Z isomers of p-nitrobenzyl 2-(1',3'-dithiolan-2'-ylmethylene)clavam-3-carboxylate (e10) (404 mg, 0.975 mmole) in dichloromethane (10 ml) was ice cooled and treated dropwise with a solution of m-chloroperbenzoic acid (1.73 g, 10 mmole) in dichloromethane (10 ml). The mixture was stirred at 0-5 °C for 4 hours, washed with saturated aqueous sodium thiosulphate solution (20 ml), 1N aqueous sodium bicarbonate solution (2 × 20 ml) and water (20 ml), dried over magnesium sulphate and evaporated under reduced pressure. The residue was chromatographed on silica (30 g) in the manner of Example 1, eluting with ethyl acetate/petrol 3 : 1 to provide a mixture of E and Z isomers of p-nitrobenzyl 2-(1',1',3',3'-tetraoxido-1',3'-dithiolan-2'-ylmethylene)clavam-3-carboxylate (e12) (191 mg, 41 %) as a white solid. $[\alpha]_D^{20}$ + 3.2° (c. 1.4 ; DMSO) ; $\nu_{max}$ ($CHCl_3$) 1,810, 1,755 and 1,680 $cm^{-1}$ ; δ ($d_6$-DMSO) 3.14 and 3.25 (1H, 2d, J 17 Hz, 6β-CH), 3.5-4.3 (5H, m, 6α-CH and —$SO_2CH_2CH_2SO_2$—), 4.70 and 4.99 (1H, 2dd, J 12 and 1.5 Hz, 8-CH), 5.1-6.1 (5H, m, —$OCH_2Ar$, 9-CH, 3-CH and 5-CH), 7.63 and 7.67 (2H, 2d, 2 Ar-H), 8.20 and 8.23 (2H, 2d, 2Ar-H).

Example 12

Mixture of E and Z isomers of lithium 2-(1',1',3',3'-tetraoxido-1',3'-dithiolan-2'-ylmethylene)clavam-3-carboxylate

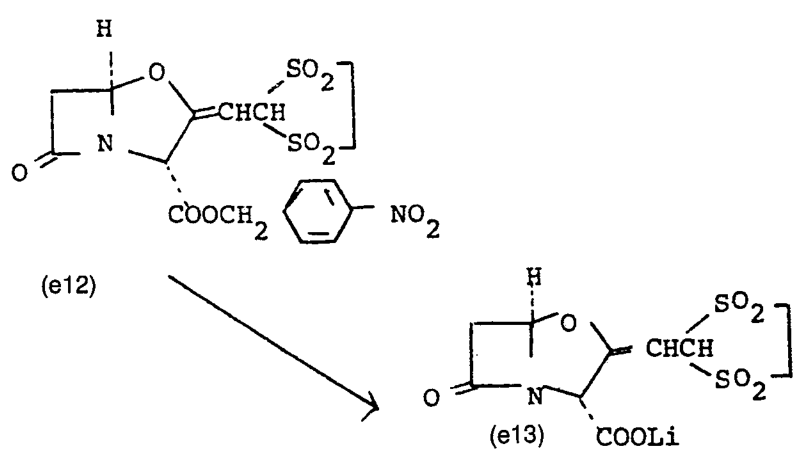

(e12)

(e13)

A solution of a mixture of E and Z isomers of p-nitrobenzyl 2-(1′,1′,3′,3′-tetraoxido-1′,3′-dithiolan-2′-ylmethylene)clavam-3-carboxylate (e12) (160 mg, 0.340 mmole) in tetrahydrofuran (5 ml) was added to a pre-hydrogenated suspension of 10% Pd/c (240 mg) in tetrahydrofuran (5 ml). Hydrogenolysis was carried out for 45 minutes at one atmosphere pressure and the mixture filtered through celite. The filtrate was evaporated under reduced pressure to 2 ml, treated with water (5 ml) and 0.1M aqueous lithium carbonate solution (1.7 ml), and washed with ethyl acetate (3 × 10 ml). The aqueous solution was adjusted to pH7 with 0.1N hydrochloric acid and evaporated under reduced pressure. The residue was chromatographed on cellulose in the manner of Example 4 to provide a mixture of E and Z isomers of lithium 2-(1′,1′,3′,3′-tetraoxido-1′,3′-dithiolan-2-ylmethylene)clavam-3-carboxylate (e13) (60 mg, 52%) as a yellow solid. NMR spectroscopy showed the geometric isomers to be present in a ratio of about 4 : 1. $[\alpha]_D^{20}$ – 18.6° (c. 0.9 ; $H_2O$) ; $\nu_{max}$ (KBr) 1,790, 1,680 and 1,620 cm$^{-1}$ ; δ ($d_6$-DMSO) 2.91 (minor) and 3.03 (major) (1H, 2d, J 17 Hz, 6β-CH), 3.4-4.3 (5H, m, 6α-CH and —$SO_2CH_2CH_2SO_2$—), 4.50 (minor) and 4.59 (major) (1H, 2dd, J 10 and 1 HZ, 8-CH), 4.78 (minor) and 4.83 (major) (1H, 2d, J 1 Hz, 3-CH), 5.43 (minor) and 6.57 (major) (1H, 2d, J 10 Hz, 9-CH, exchanges with $D_2O$), 5.73 (1H, d, J 2.5 Hz, 5-CH).

## Example 13

Benzyl 2-E-(1′,1′,3′,3′-tetraoxido-1′,3′-dithiolan-2′-ylmethylene)clavam-3-carboxylate and benzyl 2-Z-(1′,1′,3′,3′-tetraoxido-1′,3′-dithiolan-2′-ylmethylene)clavam-3-carboxylate

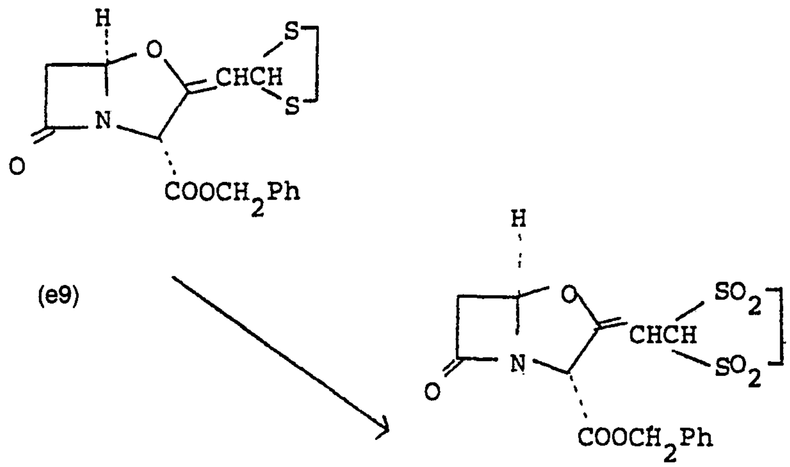

(e9)

(e14) and (e15)

A solution of a mixture of E and Z isomers of benzyl 2-(1′,3′-dithiolan-2′-ylmethylene)clavam-3′-carboxylate (e9) (290 mg, 0.80 mmole) in dichloromethane (5 ml) was ice cooled and treated dropwise with a solution of m-chloro perbenzoic acid (2.21 g, 12.8 mmole) in dichloromethane (15 ml). The mixture was stirred at 0-5 °C for 4 hours, washed with saturated aqueous sodium thiosulphate solution (20 ml), 1N aqueous sodium bicarbonate solution (2 × 20 ml) and water (20 ml), dried over magnesium sulphate and evaporated under reduced pressure. The residue was chromatographed on silica (20 g) in the manner described in Example 1, eluting with ethyl acetate/petrol (40% ethyl acetate) to produce benzyl 2-E-(1′,1′,3′,3′-tetraoxido-1′,3′-dithiolan-2-ylmethylene)clavam-3-carboxylate and benzyl 2-Z-(1′,1′,3′,3′-tetraoxido-1′,3′-dithiolan-2′-ylmethylene)clavam-3-carboxylate.

Less polar isomer (e14) (colourless gum, 44 mg, 13%) : $\nu_{max}$ ($CHCl_3$) 1,810, 1,750 and 1,680 cm$^{-1}$ ; δ ($CDCl_3$) 3.11 (1H, d, J 17 Hz, 6β-CH), 3.49 (1H, dd, J 17 and 2.5 Hz, 6α-CH), 3.68 (4H, broad s, —$SO_2CH_2CH_2SO_2$—), 5.0-5.5 (5H, m, —$OCH_2Ph$, 3-CH, 8-CH and 9-CH), 5.76 (1H, d, J 2.5 Hz, 5-CH), 7.35 (5H, s, Ph-H).

More polar isomer (e15) (colourless gum, 30 mg, 9%) : $\nu_{max}$ ($CHCl_3$) 1,810, 1,755 and 1,685 cm$^{-1}$ ; δ ($CDCl_3$) 3.14 (1H, d, J 17 Hz, 6β-CH), 3.53 (1H, dd, J 17 and 2.5 Hz, 6α-CH), 3.71 (4H, broad s, —$SO_2CH_2CH_2SO_2$—), 4.76 (1H, broad d, J 10 Hz, 8-CH), 5.07 (1H, d, J 10 Hz, 9-CH), 5.21 (2H, s, —$OCH_2Ph$), 5.31 (1H, broad s, 3-CH), 5.79 (1H, d, J 2.5 Hz, 5-CH), 7.35 (5H, s, Ph-H).

## Example 14

Lithium 2-(1′,1′,3′,3′-tetraoxido-1′,3′-dithiolan-2′-ylmethylene)clavam-3-carboxylate (less polar isomer)

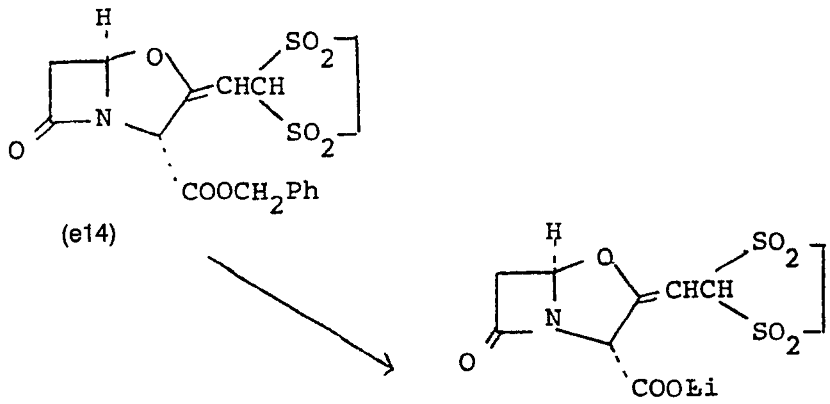

(e14)

(e16)

A solution of benzyl 2-(1′,1′,3′,3′-tetraoxido-1′,3′-dithiolan-2′-ylmethylene)clavam-3-carboxylate (e14) (less polar product of Example 13, 40 mg, 0.094 mmole) in tetrahydrofuran (2 ml) was added to a prehydrogenated suspension of 10 % Pd/c (80 mg) in tetrahydrofuran (4 ml). Hydrogenolysis of the mixture was carried out at atmospheric pressure for 5 hours and the resulting suspension was filtered through celite. The filtrate was treated with water (8 ml) and 0.1N aqueous lithium hydroxide solution to pH7. Organic solvent was evaporated at reduced pressure and the aqueous solution filtered through celite and freeze dried to produce lithium 2-(1′,1′,3′,3′-tetraoxido-1′,3′-dithiolan-2′-ylmethylene)clavam-3-carboxylate (e16) (less polar isomer on silica t. l. c., eluent chloroform/acetone/acetic acid 50 : 50 : 7, 28 mg, 88 %) as a pale yellow solid $[\alpha]_D^{20}$ – 23° (c. 1.0 ; $H_2O$) ; $\nu_{max}$ (KBr) 1,795, 1,675 and 1,620 $cm^{-1}$ ; δ ($D_2O$—$CH_3CN$=2.00) 3.11 (1H, d, J 17.5 Hz, 6β-CH), 3.56 (1H, dd, J 17.5 and 3 Hz, 6α-CH), 3.8-4.2 (4H, m, —$SO_2CH_2CH_2SO_2$—), 5.23 (1H, d, J 1 Hz, 3-CH), 5.79 (1H, d, J 3Hz, 5-CH).

Example 15

Lithium 2-(1′,1′,3′,3′-tetraoxido-1′,3′-dithiolan-2′-ylmethylene)clavam-3-carboxylate (more polar isomer)

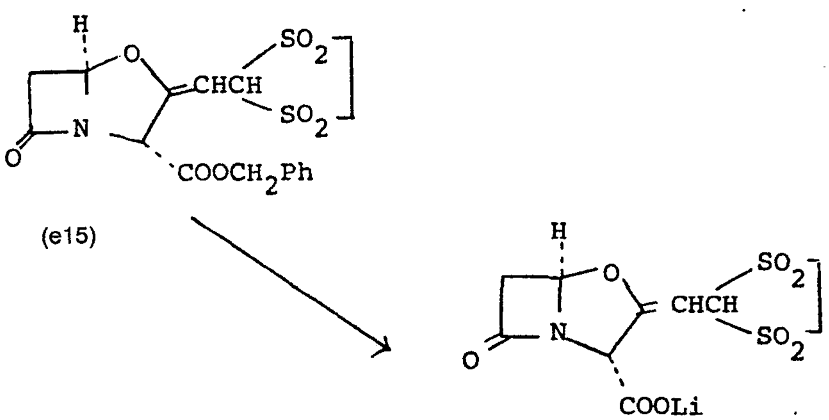

(e15)

(e17)

A solution of benzyl 2-(1′,1′,3′,3′-tetraoxido-1′,3′-dithiolan-2′-ylmethylene)clavam-3-carboxylate (e15) (more polar product of Example 13, 30 mg, 0.070 mmole) in tetrahydrofuran (2 ml) was added to a prehydrogenated suspension of 10 % Pd/c (30 mg) in tetrahydrofuran (4 ml). Hydrogenolysis was carried out for 30 minutes at atmospheric pressure and the mixture filtered through celite. The filtrate was evaporated to 2 ml, treated with water (5 ml) and 0.1N aqueous lithium hydroxide solution to pH7. Organic solvent was evaporated at reduced pressure and the aqueous solution filtered through celite and freeze dried to produce lithium 2-(1′,1′,3′,3′-tetraoxido-1′,3′-dithiolan-2′-ylmethylene)clavam-3-carboxylate (e17) (more polar isomer on silica t. l. c., eluent chloroform/acetone/glacial acetic acid 50 : 50 : 7, 20 mg, 83 %) as a yellow solid. $[\alpha]_D^{20}$ – 64.5° (c. 0.8 ; $H_2O$) ; $\nu_{max}$ (KBr) 1,785, 1,680 and 1,625 $cm^{-1}$ ; δ ($D_2$—$CH_3CN$=2.00) 3.14 (1H, d J 17 Hz, 6β-CH), 3.57 (1H, dd, J 17 and 2.5 Hz, 6α-CH), 3.9-4.2 (4H, m, —$SO_2CH_2CH_2SO_2$—), 5.15 (1H, broad, s, 3-CH), 5.80 (1H, d, J 2.5 Hz, 5-CH).

Example 16

Mixture of E and Z isomers of p-nitrobenzyl 2-(1′-oxido-1′,3′-dithiolan-2′-ylmethylene)clavam-3-carboxylate

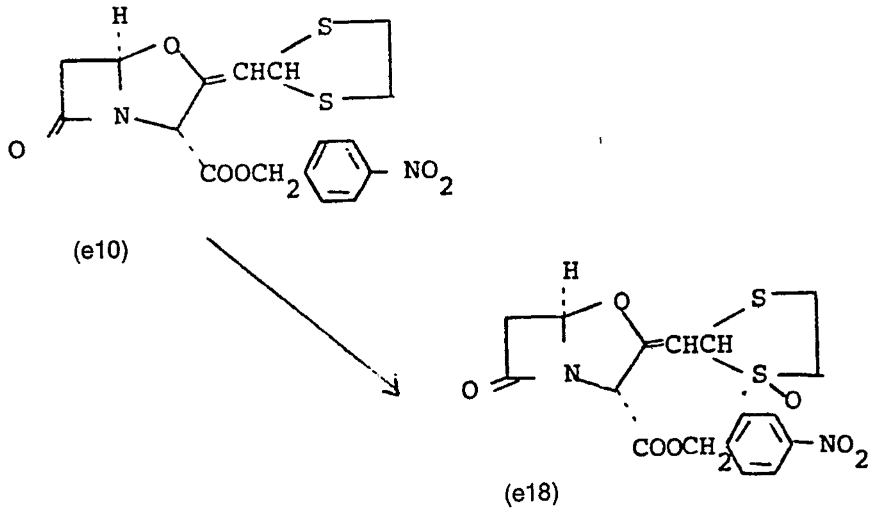

(e10)

(e18)

A solution of a mixture of E and Z isomers of p-nitrobenzyl 2-(1′,3′-dithiolan-2′-ylmethylene)clavam-3-carboxylate (e10) (185 mg, 0.45 mmole) in dichloromethane (5 ml) was ice cooled and treated dropwise with a solution of m-chloroperbenzoic acid (86 mg, 0.50 mmole) in dichloromethane (2 ml). The mixture was stirred at 0-50 °C for 1 hour, washed with 1N aqueous sodium bicarbonate solution (2 × 10 ml) and water (10 ml), dried over magnesium sulphate and evaporated under reduced pressure. The residue was chromatographed on silica as described in Example 1, eluting with ethyl acetate/petrol 3 : 1 to provide a mixture of E and Z isomers of p-nitrobenzyl 2-(1′-oxido-1′,3′-dithiolan-2′-ylmethylene)clavam-3-carboxylate (e18) (98 mg, 51 %) as a colourless gum. Besides being a mixture of geometric isomers, the product was also a mixture of diastereoisomers and a t. l. c. on silica (eluent ethyl acetate) showed 4 zones between $R_f$ 0.28 and 0.51. $\nu_{max}$ ($CHCl_3$) 1,805, 1,755, 1,680 and 1,030 $cm^{-1}$.

## Example 17

p-Nitrobenzyl 2-E-(2′,2′-diphenylthioethylidene)clavam-3-carboxylate and p-nitrobenzyl 2-Z-(2′,2′-diphenyl thioethylidene)clavam-3-carboxylate

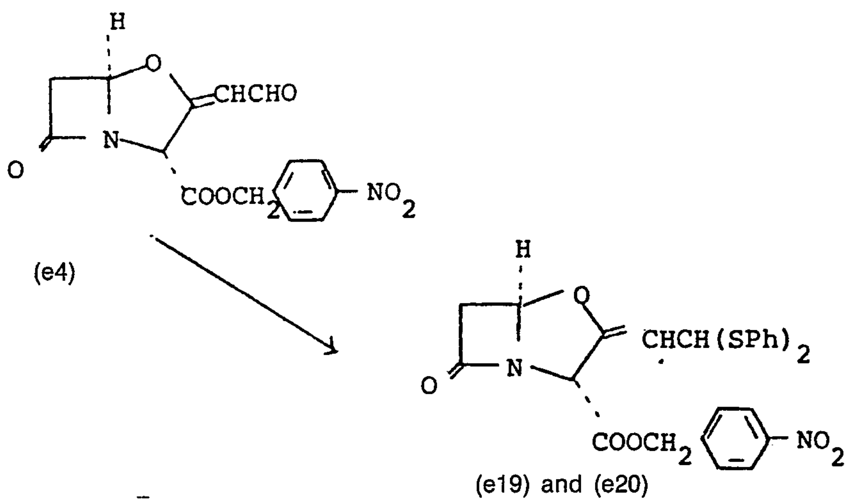

(e4)

(e19) and (e20)

A mixture of E and Z isomers of p-nitrobenzyl 2-(2′-oxoethylidene)clavam-3-carboxylate (e4) (680 mg, 2.04 mmole) was dissolved in a mixture of dichloromethane (4 ml) and thiophenol (3 ml), ice cooled and treated with borontrifluoride diethyletherate (3 drops). The mixture was stirred at 0-5 °C for 1 1/4 hours, diluted with dichloromethane (50 ml) washed with 1N aqueous sodium bicarbonate solution (30 ml), dried over anhydrous magnesium sulphate and run onto a silica column (40 g silica). Chromatography was carried out as described in Example 1, eluting with ethyl acetate/petrol 1 : 4 to provide p-nitrobenzyl 2-E-(2′,2′-diphenylthioethylidene)clavam-3-carboxylate and p-nitrobenzyl 2-Z-(2′,2′-diphenylthioethylidene)clavam-3-carboxylate.

Less polar isomer (e19) (colourless gum, 114 mg, 10.5 % : $[\alpha]_D^{20}$ – 59.0° (c. 1.1 ; $CHCl_3$) ; $\nu_{max}$ ($CHCl_3$) 1,805, 1,755 and 1,680 $cm^{-1}$ ; δ ($CDCl_3$) 2.91 (1H, d, J 17 Hz, 6β-CH), 3.39 (1H, dd, J 17 and 2.5 Hz,

6α-CH), 4.77 (1H, d, J 1.5 Hz, 3-CH), 4.9-5.4 (4H, m, —$OCH_2Ar$, 8-CH and 9-CH), 5.61 (1H, d, J 2.5 Hz, 5-CH), 7.1-7.6 12H, m, Ph-H and 2 nitrobenzyl-H), 8.14 (2H, d, 2 nitrobenzyl-H).

More polar isomer (e20) (colourless gum, 137 mg, 12.5 %) : $[\alpha]_D$ + 17.4° (c. 1.0 ; $CHCl_3$) ; $\nu_{max}$ ($CHCl_3$) 1,805, 1,755 and 1,685 $cm^{-1}$ ; δ ($CDCl_3$) 2.63 (1H, d, J 17 Hz, 6β-Ch), 3.32 (1H, dd, J 17 and 2.5 Hz, 6α-CH), 4.74 (1H, dd, J 10.5 and 1.5 Hz, 8-CH), 5.00 (1H, d, J 1.5 Hz, 3-CH), 5.16 (2H, s, —$OCH_2Ar$), 5.32 (1H, d, J 10.5 Hz, 9-CH), 5.43 (1H, d, J 2.5 Hz, 5-CH), 7.1-7.6 (12H, m, Ph-H and 2 p-nitrobenzyl-H), 8.19 (2H, d, 2 p-nitrobenzyl-H).

## Example 18

Lithium 2-(2',2'-diphenylthioethylidene)clavam-3-carboxylate (less polar isomer)

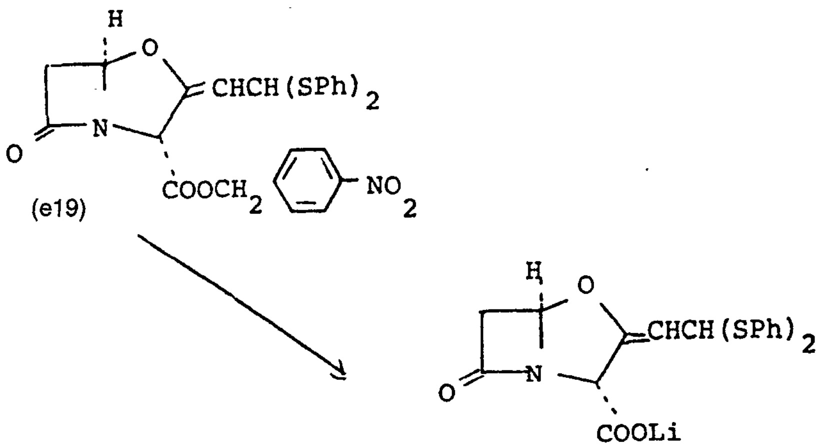

(e19)

(e21)

A solution of p-nitrobenzyl 2-(2',2'-diphenylthioethylidene)clavam-3-carboxylate (less polar product of Example 17, 43 mg, 0.080 mmole) in tetrahydrofuran (2 ml) was added to a pre-hydrogenated suspension of 10 % Pd/c (65 mg) in tetrahydrofuran (4 ml). Hydrogenolysis was carried out for 1 1/2 hours, the suspension filtered through celite and the filtrate evaporated to 2 ml under reduced pressure. This solution was ice cooled, treated with water (5 ml) and 0.1M aqueous lithium carbonate solution (0.81 ml) and washed with ether (3 × 10 ml). The aqueous solution was adjusted to pH 7 with 0.1N hydrochloric acid and evaporated under reduced pressure. The residue was chromatographed as described in Example 4 to provide lithium 2-(2',2'-diphenylthioethylidene)clavam-3-carboxylate (less polar isomer in silica t. l. c., eluent chloroform/acetone/glacial acid 50 : 50 : 7, 20 mg, 62 %) as a buff solid. $\nu_{max}$ (KBr) 1,785, 1,675 (s) and 1,625 $cm^{-1}$ ; δ ($D_2O$—HOD≡4.60( 2.68 (1H, d, J 17 Hz, 6β-CH), 3.24 (1H, dd, H 17 and 2.5 Hz, 6α-CH), 4.82 (1H, dd, J 11.5 and 1.5 Hz, 8-CH), 5.27 (1H, d, J 11.5 Hz, 9-CH), 5.43 (1H, d, J 2.5 Hz, 5-CH), 7.1-7.5 (10H, m, Ph-H).

## Example 19

Lithium 2-(2',2'-diphenylthioethylidene)clavam-3-carboxylate (more polar isomer)

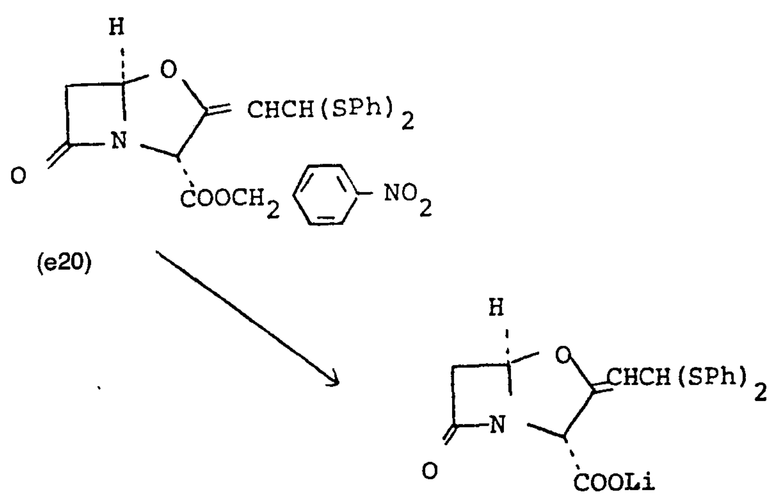

(e20)

(e22)

A solution of p-nitrobenzyl 2-(2',2'-diphenyl thioethylidene)clavam-3-carboxylate (more polar product of Example 17, 65 mg, 0.122 mmole) in tetrahydrofuran (2 ml) was added to a pre-hydrogenated suspension of 10 % Pd/c (100 mg) in tetrahydrofuran (5 ml). Hydrogenolysis was carried out for 1 1/2 hours, the suspension filtered through celite and the filtrate evaporated to 2 ml under reduced pressure. The solution was ice cooled, treated with water (5 ml) and 0.1M aqueous lithium carbonate solution (1.22 ml) and washed with ether (3 × 10 ml). The aqueous solution was adjusted to pH7 with 0.1N hydrochloric acid and evaporated under reduced pressure. The residue was chromatographed as described in Example 4 to provide lithium 2-(2',2'-diphenylthioethylidene) clavam-3-carboxylate (more polar isomer on silica t. l. c., eluent chloroform/acetone/glacial acetic acid 50 : 50 : 7, 20 mg, 41 %) as a yellow solid. $\nu_{max}$ (KBr), 1,785, 1,680 and 1,620 $cm^{-1}$ ; δ ($D_2O$—HOD ≡ 4.60) 2.48 (1H, d, J 17 Hz, 6β-CH), 3.21 (1H, dd, J 17 and 2.5 Hz, 6α-CH), 5.25 (1H, d, J 2.5 Hz, 5-CH), 7.0-7.5 (10H, m, Ph-H).

## Preparation 1

### Benzyl 2-(2'-oxoethylidene)clavam-3-carboxylate

This compound is described by Corbett, Howarth and Stirling, JCS, Chem Comm (1977) 808.

Pyridinium chlorochromate (5.46 g) and powdered, anhydrous sodium acetate (0.37 g) were suspended in dichloromethane (150 ml), ice colled and stirred vigorously. A solution of benzyl clavulanate (4.34 g) in dichloromethane (10 ml) was added and after 2 minutes the ice bath was removed. The mixture was stirred 45 minutes at room temperature, ethyl acetate (200 ml) added and the suspension filtered through celite. The filtrate was evaporated and the residue taken up in ethyl acetate/petrol 1 : 1 and filtered through a 4 cm thick pad of silica (Merck Kieselgel 60, 230-400 mesh). The filtrate was evaporated and the residue chromatographed rapidly on silica (50 g in a 4 cm diameter column) to provide benzyl 2-(2'-oxoethylidene)clavam-3-carboxylate (750 mg) as a mixture of E and Z isomers : $\nu_{max}$ ($CHCl_3$) 1,810, 1,750, 1,675, 1,650 and 1,605 $cm^{-1}$.

## Preparation 2

### Benzyl 2-(2'-oxoethylidene)clavam-3-carboxylate

(a) Benzyl 9-0-pyruvyl clavulanate

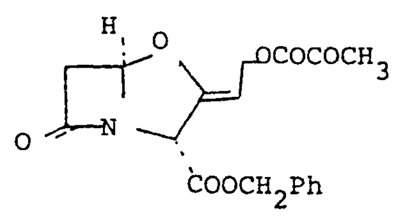

Benzyl clavulanate (2.89 g, 0.01 mole) was dissolved in dichloromethane (20 ml) and pyridine (0.81 ml, 0.01 mole), cooled to − 30 °C and treated dropwise with a solution of pyruvyl chloride (1.07 g, 0.01 mole) in dichloromethane (5 ml). The mixture was stirred for 30 minutes, washed with 0.5N hydro chloric acid (20 ml) and water (20 ml), dried over magnesium sulphate and evaporated under reduced pressure. The residue was chromatographed on silica, eluting with ethyl acetate/petrol (30 % ethyl acetate) to provide benzyl 9-0-pyruvyl clavulanate (1.70 g, 47 %) as a colourless gum. $[\alpha]_D^{20}$ + 41.4° (c. 1.1 ; $CHCl_3$) ; $\nu_{max}$ ($CHCl_3$) 1,810, 1,750 (s), 1,740 and 1,700 $cm^{-1}$ ; δ ($CDCl_3$) 2.41 (3H, s, —$COCH_3$), 3.04 (1H, d, J 17 Hz, 6β-CH), 3.48 (1H, dd, J 17 and 3 Hz, 6α-CH), 4.7-5.0 (3H, m, 8-CH and 9-$CH_2$), 5.09 (1H, s, 3-CH), 5.17 (2H, s, —$OCH_2Ph$), 5.71 (1H, d, J 3 Hz, 5-CH), 7.33 (5H, s, Ph-H).

(b) Benzyl 2-(2'-oxoethylidene)clavam-3-carboxylate

Benzyl 9-0-pyruvyl clavulanate (100 mg, 0,28 mmole) was dissolved in dry benzene (200 ml) in a quartz vessel and dry nitrogen bubbled through the solution for 11/2 hours. The nitrogen flow was continued and the vessel water cooled while being irradiated from a Hanovia medium pressure UV lamp for 45 minutes. T. l. c. (silica, eluent ethyl acetate/petrol 1 : 1) indicated complete conversion of benzyl 9-0-pyruvyl clavulanate to benzyl 2-(2'-oxoethylidene)clavam-3-carboxylate. The solution was evaporated under reduced pressure and the residue chromatographed rapidly on silica (5 g) to provide benzyl 2-(2'-oxoethylidene)clavam-3-carboxylate (50 mg, 62 %) as a mixture of E and Z isomers.

## Preparation 3

### p-Nitrobenzyl 2-(2'-oxoethylidene)clavam-3-carboxylate

Pyridinium chlorochromate (4.85 g) and powdered anhydrous sodium acetate (0.37 g) were suspended in dichloromethane (150 ml), ice cooled and stirred vigorously. A solution of p-nitrobenzyl clavulanate (5 g) in dichloromethane (20 ml) was added and after 2 minutes the ice bath was removed. The mixture was stirred for 45 minutes at room temperature and worked up exactly as described in preparation 1 to provide p-nitrobenzyl 2-(2'-oxoethylidene)clavam-3-carboxylate (600 mg) as a yellow oil (mixture of E and Z isomers). $\nu_{max}$ ($CHCl_3$) 1,810, 1,750, 1,670, 1,645 and 1,605 $cm^{-1}$.

Preparation 4

p-Nitrobenzyl 2-(2'-oxoethylidene)clavam-3-carboxylate

(a) p-Nitrobenzyl 9-0-pyruvyl clavulanate

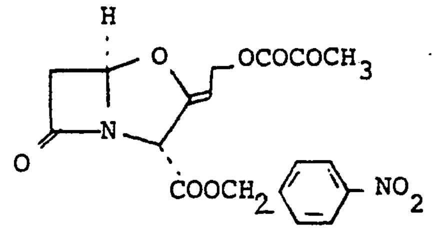

P-Nitrobenzyl clavulanate (2.0 g, 6 mmole) was dissolved in dichloromethane (20 ml) and pyridine (0.507 ml, 6.3 mmole), cooled to − 30 °C and treated dropwise with a solution of pyruvyl chloride (0.67 g, 6.3 mmole) in dichloromethane (5 ml). The solution was stirred 30 minutes, washed with 0.5N hydrochloric acid (20 ml) and water (20 ml), dried over magnesium sulphate and evaporated under reduced pressure. The residue was chromatographed on silica, eluting with ethyl acetate/petrol (40 % ethyl acetate) to provide p-nitrobenzyl 9-0-pyruvyl clavulanate (1.2 g, 50 %) as a pale yellow gum. $\nu_{max}$ ($CHCl_3$) 1,810, 1,755, 1,735 and 1,700 $cm^{-1}$; δ ($CDCl_3$) 2.44 (3H, s, $-COCH_3$), 3.11 (1H, d, J 17 Hz, 6β-CH), 3.54 (1H, dd, J 17 and 2.5 Hz, 6α-CH), 4.7-5.1 (3H, m, 8-CH and 9-$CH_2$), 5.14 (1H, s, 3-CH), 5.28 (2H, s, $-OCH_2Ar$), 5.73 (1H, d, J 2.5 Hz, 5-CH), 7.51 and 8.24 (4H, 2d, Ar-H).

(b) p-Nitrobenzyl 2-(2'-oxoethylidene)clavam-3-carboxylate

p-Nitrobenzyl 9-0-pyruvyl clavulanate (200 mg) was dissolved in dry benzene (200 ml) in a quartz vessel and dry nitrogen bubbled through the solution for 1 hour. The nitrogen flow was continued and the vessel water cooled while being irradiated from a Hanovia medium pressure UV lamp for 4 hours. The solution was then evaporated under reduced pressure and the residue chromatographed rapidly on silica (5 g) to provide p-nitrobenzyl 2-(2'-oxoethylidene)clavam-3-carboxylate (50 mg, 30 %) as a mixture of E and Z isomers.

Biological data

Synergistic activity *in vitro* of some of the compounds of the present invention.

| Compound | Inhibitor Conc (mg/ml) | Staph aureus Russell | Klebsiella aerogenes E70 | E. coli JT 39 |
|---|---|---|---|---|
| Amoxycillin alone | - | 500 | 250-500 | ≥2000 |
| Amoxycillin with Compound of Example No 10 | 5 | 0.3 | 12.5 | 8 |
| | 1 | 0.6 | 50 | 31.2 |
| Amoxycillin with Compound of Example No 12 | 5 | 0.6 | 3.1 | 2 |
| | 1 | 1.25 | 3.1 | 8 |

(Continued)

| Compound | Inhibitor Conc (mg/ml) | Staph aureus Russell | Klebsiella aerogenes E70 | E. coli JT 39 |
|---|---|---|---|---|
| Amoxycillin with Compound of Example No 4 | 5 | 0.6 | 50 | >500 |
| | 1 | 2.5 | >100 | >500 |
| Amoxycillin with Compound of Example No 5 | 5 | 0.08 | 25 | 62.5 |
| | 1 | 0.6 | 100 | >500 |

**Claims**

1. A compound of formula (I) or a pharmaceutically acceptable salt or *in vivo* hydrolysable ester thereof :

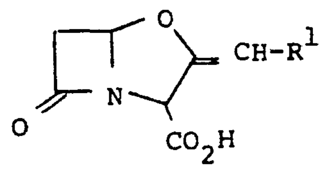

(I)

wherein $R^1$ is a group of structure (II) or (III) :

(II) 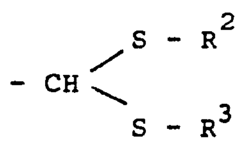

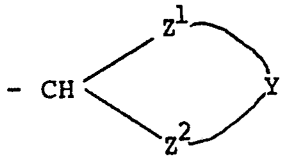 (III)

wherein $R^2$ and $R^3$ may be the same or different and each represents $C_{1-6}$ alkyl optionally substituted by $C_{1-6}$ alkoxy, phenyl, hydroxy, carboxy or $C_{1-6}$ alkoxycarbonyl, or phenyl optionally substituted by $C_{1-6}$ alkyl, halogen, $C_{1-6}$ alkoxy, hydroxy, carboxy or $C_{1-6}$ alkoxycarbonyl ; $Z^1$ and $Z^2$ may be the same or different and each represents S, SO or $SO_2$ and Y represents a $C_{2-3}$ saturated hydrocarbon radical optionally substituted by $C_{1-6}$ alkyl, $C_{1-6}$ alkoxy, hydroxy, carboxy, or $C_{1-6}$ alkoxycarbonyl.

2. A compound as claimed in claim 1 wherein $R^2$ and $R^3$ may be the same or different and each represents methyl, ethyl, n- or iso-propyl, n-, sec-, tert- or iso-butyl, benzyl or phenyl.

3. A compound as claimed in claim 1 or claim 2 wherein $R^2$ and $R^3$ are the same.

4. A compound as claimed in claim 1 of formula (IV) or a pharmaceutically acceptable salt or *in vivo* hydrolysable ester thereof :

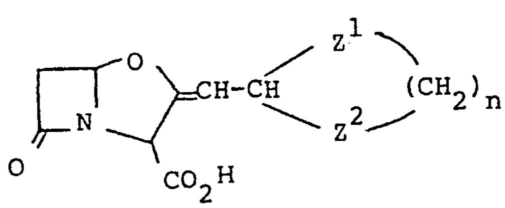

(IV)

wherein $Z^1$ and $Z^2$ are as defined hereinbefore with respect to formula (I), and n is an integer from 2 to 3.

5. A compound as claimed in claim 4 wherein n is 2.

6. A compound as claimed in claim 4 or claim 5 wherein $Z^1$ and Z are the same and represent S or $SO_2$.

7. A compound as claimed in claim 6 wherein $Z^1$ and $Z^2$ are the same and represent $SO_2$.

8. A process for the preparation of a compound of formula (I) or a pharmaceutically acceptable salt or *in vivo* hydrolysable ester thereof which process comprises reacting a compound of formula (V) :

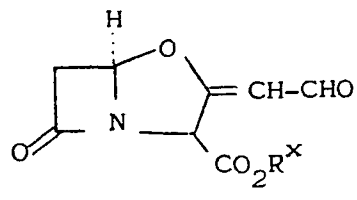

(V)

wherein $R^x$ is a carboxyl blocking group ;

(1) with compound $R^2SH$ and $R^3SH$ wherein $R^2$ and $R^3$ are as defined with respect to formula (I) and wherein any reactive groups may be protected ; or

(2) with a compound of formula HS—Y—SH wherein Y is as defined with respect to formula (I) and wherein any reactive groups may be protected ; in the presence of a Lewis acid catalyst : and thereafter where necessary carrying out one or more of the following steps :

(a) oxidising a sulphide to a sulphoxide or sulphone derivative ;

(b) removing the carboxy blocking group $R^x$ ;

(c) removing any protecting groups on $R^2$ or $R^3$ or Y ; and

(d) converting the compound to a free carboxylic acid, a pharmaceutically acceptable salt or *in vivo* hydrolysable ester.

9. A pharmaceutical composition which comprises a compound as claimed in any one of claims 1 to 7 together with a pharmaceutically acceptable carrier.

10. A compound as claimed in claim 1 for use in treating bacterial infections in humans or domestic mammals.

**Ansprüche**

1. Eine Verbindung der Formel (I) oder ein pharmakologisch annehmbares Salzes oder ein *in vivo* hydrolysierbarer Ester derselben

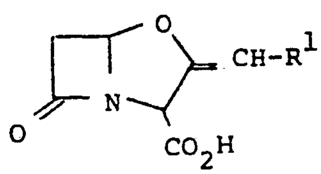

(I)

in welcher $R^1$ eine Gruppe der Struktur (II) oder (III) bedeutet :

(II) 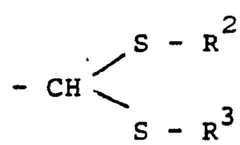

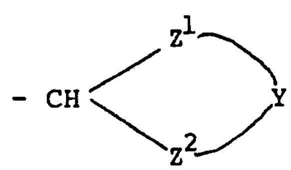 (III)

wobei $R^2$ und $R^3$ gleich oder verschieden sein können und jeweils $C_{1-6}$-Alkyl-, gegebenenfalls substituiert durch $C_{1-6}$-Alkoxy, Phenyl, Hydroxy, Carboxy oder $C_{1-6}$-Alkoxycarbonyl oder Phenyl, gegebenenfalls substituiert durch $C_{1-6}$-Alkyl, Halogen, $C_{1-6}$-Alkoxy, Hydroxy, Carboxy oder $C_{1-6}$-Alkoxycarbonyl ist ; $Z^1$ und $Z^2$ gleich oder verschieden sein können und jeweils S, SO oder $SO_2$ bedeuten und Y einen gesättigten $C_{2-3}$-Kohlenwasserstoffrest darstellt, der gegebenenfalls durch $C_{1-6}$-Alkyl, $C_{1-6}$-Alkoxy, Hydroxy, Carboxy oder $C_{1-6}$-Alkoxycarbonyl substituiert ist.

2. Eine Verbindung wie in Anspruch 1 beansprucht, in welcher $R^2$ und $R^3$ gleich oder verschieden sein können und jeweils Methyl, Äthyl-, n- oder iso-Propyl, n-, sek.-, tert.- oder iso-Butyl, Benzyl oder Phenyl bedeuten.

3. Eine Verbindung wie in Anspruch 1 oder 2 beansprucht, in welcher $R^2$ und $R^3$ gleich sind.

4. Eine Verbindung wie in Anspruch 1 beansprucht, der Formel (IV) oder ein pharmakologisch annehmbares Salz oder ein *in vivo* hydrolysierbarer Ester derselben :

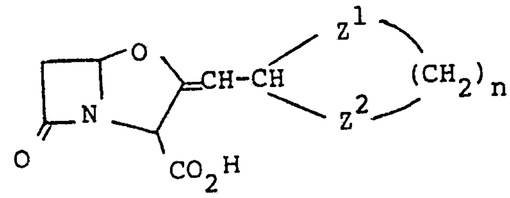

(IV)

in welcher $Z^1$ und $Z^2$ wie vorstehend in Bezug auf Formel (I) definiert sind und n eine ganze Zahl von 2 oder 3 ist.

5. Eine Verbindung wie in Anspruch 4 beansprucht, in welcher n 2 ist.

6. Eine Verbindung wie in Anspruch 4 oder 5 beansprucht, in welcher $Z^1$ und $Z^2$ gleich sind und S oder $SO_2$ bedeuten.

7. Eine Verbindung wie in Anspruch 6 beansprucht, in welcher $Z^1$ und $Z^2$ gleich sind und $SO_2$ bedeuten.

8. Ein Verfahren zur Herstellung einer Verbindung der Formel (I) oder eines pharmakologisch annehmbaren Salzes oder eines *in vivo* hydrolysierbaren Esters derselben, welches Verfahren die Umsetzung einer Verbindung der Formel (V) :

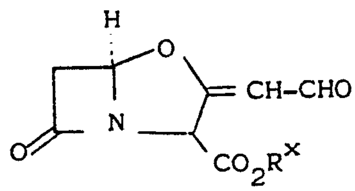

(V)

in welcher $R^x$ eine die Carboxylgruppe blockierende Gruppe bedeutet, in Anwesenheit eines Lewissäure-Katalysators

(1) mit Verbindungen $R^2SH$ und $R^3SH$, in welchen $R^2$ und $R^3$ wie in Bezug auf Formel (I) definiert sind, und in welchen etwa vorhandene reaktive Gruppen geschützt sein können oder

(2) mit einer Verbindung der Formel HS—Y—SH, in welcher Y wie in Bezug auf Formel (I) definiert ist und gegebenenfallsvorhandene reaktive Gruppen geschützt sein können, und anschließend, falls erforderlich, das Durchführen einer oder mehrerer der nachstehenden Verfahrensstufen umfaßt :

(a) Oxidieren eines Sulfids zu einem Sulfoxid- oder Sulfonderivat ;

(b) Entfernen der die Carboxylgruppe blockierenden Gruppe $R^x$ ;

(c) Entfernen etwa an $R^2$ oder $R^3$ oder Y vorhandenen Schutzgruppen ; und

(d) Umwandeln der Verbindung zu einer freien Carbonsäure, einem pharmakologisch annehmbaren Salz oder einem *in vivo* hydrolysierbaren Ester derselben.

9. Eine pharmazeutische Zusammensetzung, welche eine Verbindung wie in irgendeinem der Ansprüche 1 bis 7 beansprucht zusammen mit einem pharmakologisch annehmbaren Träger enthält.

10. Eine Verbindung wie in Anspruch 1 beansprucht zur Anwendung bei der Behandlung bakterieller Infektionen beim Menschen oder bei Haus-Säugetieren.

**Revendications**

1. Composé de formule (I) ou sel pharmaceutiquement acceptable ou ester hydrolysable *in vivo* de celui-ci

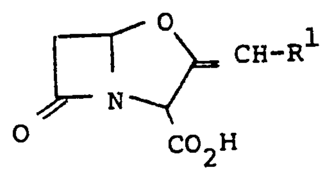

(I)

formule dans laquelle $R^1$ est un groupe de structure (II) ou (III) :

(II) 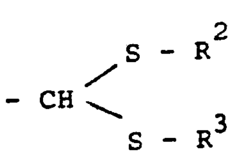

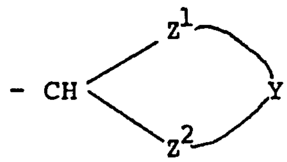 (III)

dans lesquelles $R^2$ et $R^3$ peuvent être identiques ou différents et représentent chacun un groupe alcoyle en $C_{1-6}$ facultativement substitué par un groupe alcoxy en $C_{1-6}$, phényle, hydroxy, carboxy ou alcoxy($C_{1-6}$) carbonyle, ou bien phényle facultativement substitué par un groupe alcoyle en $C_{1-6}$, halogène, alcoxy en $C_{1-6}$, hydroxy, carboxy ou alcoxy($C_{1-6}$)carbonyle ; $Z^1$ et $Z^2$ peuvent être identiques ou différents et représentent chacun S, SO ou $SO_2$ et Y représente un radical hydrocarboné saturé en C[illegible] facultativement substitué par un groupe alcoyle en $C_{1-6}$, alcoxy en $C_{1-6}$, hydroxy, carboxy ou alcoxy($C_{1-6}$) carbonyle.

2. Composé suivant la revendication 1, caractérisé en ce que $R^2$ et $R^3$ peuvent être identiques ou différents et représentent chacun un groupe méthyle, éthyle, n- ou iso-propyle, n-, sec-, t- ou iso-butyle, benzyle ou phényle.

3. Composé suivant la revendication 1 ou 2, caractérisé en ce que $R^2$ et $R^3$ sont identiques.

4. Composé suivant la revendication 1 de formule (IV) ou sel pharmaceutiquement acceptable ou ester hydrolysable *in vivo* de ce composé :

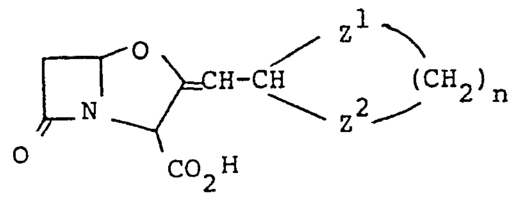 (IV)

dans laquelle $Z^1$ et $Z^2$ sont tels que définis ci-avant dans le cas de la formule (I) et n est un nombre entier allant de 2 à 3.

5. Composé suivant la revendication 4, caractérisé en ce que n est égal à 2.

6. Composé suivant la revendication 4 ou 5, caractérisé en ce que $Z^1$ et $Z^2$ sont identiques et représentent S ou $SO_2$.

7. Composé suivant la revendication 6, caractérisé en ce que $Z^1$ et $Z^2$ sont identiques et représentent $SO_2$.

8. Procédé pour la préparation d'un composé de formule (I) ou d'un sel pharmaceutiquement acceptable ou ester hydrolysable *in vivo* de celui-ci, ce procédé consistant à faire réagir un composé de formule (V) :

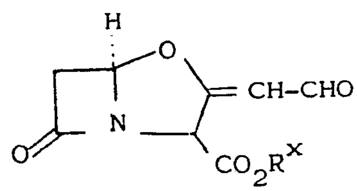 (V)

dans laquelle $R^x$ est un groupe de blocage de carboxy :

(1) avec des composés $R^2SH$ et $R^3SH$, formules dans lesquelles $R^2$ et $R^3$ sont tels que définis dans le cas de la formule (I) et les groupes réactifs peuvent être protégés ; ou

(2) avec un composé de formule HS—Y—SH dans laquelle Y est tel que défini dans le cas de la formule (I) et les groupes réactifs peuvent être protégés ; en présence d'un catalyseur formé par un acide de Lewis, puis si nécessaire à effectuer un ou plusieurs des stades opératoires ci-après

(a) oxydation d'un sulfure en un dérivé sulfoxyde ou sulfone ;

(b) élimination du groupe de blocage du carboxy $R^x$ ;

(c) élimination de tout groupe de protection sur $R^2$ ou $R^3$ ou Y ; et

(d) conversion du composé en un acide carboxylique libre, un sel pharmaceutiquement acceptable ou un ester hydrolysable *in vivo.*

9. Composition pharmaceutique renfermant un composé suivant l'une quelconque des revendications 1 à 7, conjointement à un véhicule ou excipient pharmaceutiquement acceptable.

10. Composé suivant la revendication 1, destiné à être utilisé pour le traitement des infections bactériennes chez les êtres humains ou les mammifères domestiques.